(19) Europäisches Patentamt
European Patent Office
Office européen des brevets

(11) Publication number: 0 038 708
B1

(12) # EUROPEAN PATENT SPECIFICATION

(45) Date of publication of patent specification: 24.08.83

(51) Int. Cl.³: C 07 C 109/12 //B01J23/72

(21) Application number: 81301752.2

(22) Date of filing: 21.04.81

(54) **Process for producing benzophenone-azines.**

(30) Priority: 21.04.80 JP 52612/80

(43) Date of publication of application:
28.10.81 Bulletin 81/43

(45) Publication of the grant of the patent:
24.08.83 Bulletin 83/34

(84) Designated Contracting States:
DE FR GB

(56) References cited:
GB - A - 2 051 785
US - A - 2 870 206
US - A - 4 079 080

Patents Abstracts of Japan, Vol. 3, No. 3, February 1979, page C—38

Chemical Abstracts vol. 89, no. 21, 20 November 1978, Columbus, Ohio, USA, I. ISSHIKI et al. "Benzophenone azine" page 579, right hand column, abstract no. 179707y

The file contains technical information submitted after the application was filed and not included in this specification

(73) Proprietor: MITSUBISHI GAS CHEMICAL COMPANY, INC.
5-2, Marunouchi 2-chome Chiyoda-Ku
Tokyo (JP)

(72) Inventor: Isshiki, Tomiya
14-11, Umegaoka 2-chome Setagaya-ku
Tokyo (JP)
Inventor: Sakaguchi, Shuzabu
2511-12, Oaza Fukawa Tonemachi
Kita Souma-gun Ibaraki-ken (JP)
Inventor: Kohzahi, Toshiaki
2689-20, Kashiwada Ushiku-cho
Inashiki-gun Ibaraki-ken (JP)
Inventor: Aoki, Osamu
14-6, Jinyamae Tokiwadaira
Matsudo-shi Chiba-ken (JP)
Inventor: Takeda, Norio
14-6, Jinyamae Tokiwadaira
Matsudo-shi Chiba-ken (JP)

(74) Representative: Cropp, John Anthony David et al,
MATHYS & SQUIRE 10 Fleet Street
London, EC4Y 1AY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 038 708 B1

Courier Press, Leamington Spa, England

# Process for producing benzophenone-azines

This invention relates to a process for producing a benzophenone-azine which comprises reacting a benzophenone-imine with molecular oxygen in the presence of dissolved copper in amount of 1/100—1/2000 mol per 1 mol of the benzophenone-imine in terms of copper atom.

A benzophenone-azine is useful as an intermediate for producing hydrazine from benzophenones.

Many processes for producing benzophenone-azines by oxidizing benzophenone-imines are known in the art. A process for producing a benzophenone-azine by oxidizing benzophenone-imines in the presence of a copper halide, particularly cuprous chloride is known from USP No. 2,870,206.

A process for producing a benzophenone-azine by oxidizing a benzophenone-imine in the presence of excess amount of pyridine by using copper (II) halide methoxide is known from Japanese Patent Publication (laid open) No. 147047/1978. Japanese Patent Publication (laid open) No. 71045/1978 discloses that copper acetates, copper thiocyanate, copper cyanide and the like are useful for preparing benzophenone-azines from benzophenone-imines. Japanese Patent Publication (laid open) No. 131987/1977 discloses that a high molecular catalyst obtained by coordinating cuprous halide in a resin, the functional group of which is pyridines, is useful for oxidizing benzophenone-imines.

However, in these processes an expensive copper salt in a high concentration is used. For example, US Patent No. 2,870,206 employs Cu in amount of 1 mol/4.31 mol of imine (0.23 mol Cu/1 mol imine) (Example 1) and 1 mol of Cu/13.5 mol of imine (0.07 mol Cu/1 mol imine) (Example 2). Japanese Patent Publication (laid open) No. 147047/1978 employs an insoluble solid catalyst wherein copper (I) halide or methoxy copper (II) halide is coordinated with a resin having a monodentate pyridine as a functional group. When methoxy copper halide is used, the amount of copper is 0.05 mol Cu/1 mol imine. When insoluble catalyst coordinated with a resin having a monodentate pyridine is used, the catalyst can easily be separated from the reaction mixture. However, this insoluble catalyst contains expensive resin containing pyridine and a large amount of copper halide. Furthermore, such resin is deteriorated when oxidation of benzophenone is effected. Therefore, this insoluble resin cannot be used for industrial oxidation of benzophenone. In addition, Japanese Patent Publication (laid open) No. 71045/1978 discloses the use of copper in an amount of 1 mol Cu/100 mol imine. However, the reference states that when copper in a concentration as low as 0.01 mol Cu/1 mol imine is used, the reaction rate is slow. In the actual working examples of the reference, copper chloride is used in amount which gives a ratio of Cu to imine of 1/21.5 to 1/21. Japanese Patent Publication (laid open) No. 131987/1977 discloses an insoluble catalyst wherein copper halide is coordinated with a resin having a monodentate pyridine. The invention of the reference has the same disadvantages as the invention of Japanese Patent Publication No. 147047/1978.

When copper salts are used as liquid phase homogeneous catalyst, it is necessary to control the valency of the copper, to keep the ratio of copper ion to anion constant, or to introduce a ligand, such as expensive pyridine into the catalyst for keeping the reaction activity of the catalyst. In addition, since the prior catalyst is dissolved in the reaction solution, the prior processes need complicated operations to recover copper salt as a catalyst from the solution. For example, operations, such as separation of the copper salt from the solution and regeneration of the copper catalyst, etc. are necessary. So, the cost of equipment increases and a loss of energy is incurred in operation. Further, expensive anticorrosive material should be used for the equipment in order to prevent corrosion of the equipment by the halogen constituting the catalyst. Accordingly, the processes are unsuitable from an industrial point of view.

When high molecular catalyst coordinating copper ion is used, copper ion is released from the catalyst during the reaction and the copper ion is not completely reabsorbed even at the end of the reaction. In addition, special resins having unidentate pyridine as functional group must be used. Therefore, the process is not satisfactory from an industrial point of view.

We carried out research for overcoming the above short-coming and found that a catalyst selected from the group consisting of transition elements belonging to the fourth, fifth and sixth periods of the Periodic Table, typical elements belonging to the fifth and sixth periods of the Periodic Table and compounds of these metals are effective for converting a benzophenone imine to the corresponding benzophenone-azine. We also found metallic copper to be excellent for converting a benzophenone-imine to the corresponding benzophenone-azine with respect to selectivity and activity (see British patent application 8015492, now GB—A—2 051 785, of which Examples 20 to 55 illustrate the use of various forms of copper, copper alloy and copper oxide) and at the time of that discovery considered that the active catalyst was undissolved copper, undissolved copper alloy or undissolved copper compound. We have since carried out further research to find a better catalyst. As a result, unexpectedly, we found that a small amount of dissolved copper is a useful catalyst for producing benzophenone-azines from benzophenone-imines.

Accordingly, the present invention provides a process for producing a benzophenone-azine having the formula

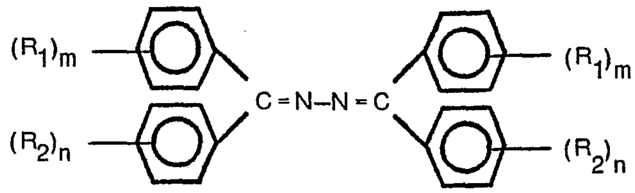

I

wherein $R_1$ and $R_2$ may be the same or different, and each of $R_1$ and $R_2$ is independently aliphatic, alicyclic or aromatic hydrocarbon having 1—10 carbon atoms,

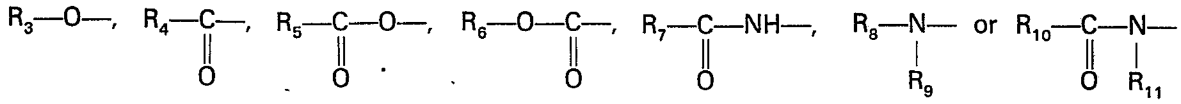

wherein $R_3$, $R_4$, $R_5$, $R_6$, $R_7$, $R_8$, $R_9$, $R_{10}$ and $R_{11}$ are independently alkyl having 1—10 carbon atoms; halogen; hydroxy; nitro; or cyano; or $R_1$ and $R_2$ when taken together form a single bond or substituted or unsubstituted methylene wherein the substituent is methyl; m is 0 or integer of 1—5; and n is 0 or integer of 1—5, which comprises reacting a benzophenone-imine having the formula

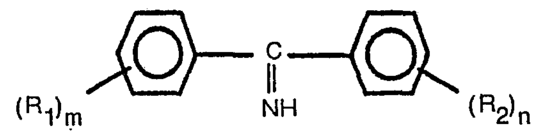

II

wherein $R_1$, $R_2$, m and n are as defined above with molecular oxygen in the presence of a catalyst composed of dissolved copper in an amount of 0.0005—0.01 mol per 1 mol of the benzophenone-imine in terms of copper atom, wherein the dissolved copper is obtained by dissolving in the benzophenone-imine at least one copper-containing material selected from metallic copper, copper alloys, copper oxides, copper hydroxide, copper carbonate, copper sulfide, formic acid salt of copper, salts of phenols and copper, and mixtures thereof.

The dissolved copper is believed to be present in the form of complex. However, the chemical structure of the complex can not be specified, because the benzophenone-imine solution contains various compounds, depending on processes for producing benzophenone-imines and on copper salts employed as a copper source.

Benzophenone-imines are represented by formula II. Examples of benzophenone-imines employed in the practice of this invention include benzophenone-imine, 2-, 3- or 4-methylbenzophenone-imine, 2-, 3- or 4-ethylbenzophenone-imine, 2-, 3- or 4-n-propylbenzophenone-imine, 2-, 3- or 4-propylbenzophenone-imine, 2-, 3- or 4-n-butylbenzophenone-imine, 2-, 3- or 4-iso-butylbenzophenone-imine, 2-, 3- or 4-tert.-butylbenzophenone-imine, 2-, 3- or 4-amylbenzophenone-imine, 2-, 3- or 4-decylbenzophenone-imine, 2-, 3- or 4-methoxybenzophenone-imine, 4-cyclohexyl-benzophenone-imine, 4-phenylbenzophenone-imine, 2,4-dimethylbenzophenone-imine, 2,3-dimethyl-benzophenone-imine, 3, 4-dimethylbenzophenone-imine, 2,4-diethylbenzophenone-imine, 2,3-diethylbenzophenone-imine, 3,4-diethylbenzophenone-imine, 2-methyl-4-ethylbenzophenone-imine, 2-methyl-4-butylbenzophenone-imine, 2,2′-, 3,3′-, 4,4′-, 2,3′-, 2,4′- or 3,4′-dimethylbenzophenone-imine, 2-, 3- or 4-chlorobenzophenone-imine, 2-chloro-4-methylbenzophenone-imine, 4-chloro-4′-methylbenzophenone-imine, 4,4′-dichlorobenzophenone-imine, 4-nitrobenzophenone-imine, 2,4-dinitrobenzophenone-imine, 4-hydroxybenzophenone-imine, 4-N,N-dimethylaminobenzophenone-imine, 4-actylbenzophenone-imine, 4-methoxycarbonylbenzophenone-imine, 4-hydroxybenzophenone-imine, 4-cyanobenzophenone-imine, fluorenone-imine, anthron-imine and 10-methyl anthron-imine. Other benzophenones according to the formula given in claim 1 are also usable in the practice of this invention.

The benzophenone-imines represented by formula II may be prepared by reacting the corresponding benzophenones with ammonia by reacting benzonitrile with aryl magnesium bromide or by dehydrating diaryl aminoalcohol. The benzophenone-imines prepared by any one of these processes can be used in the present invention as a raw material.

Benzophenone-imine and benzophenone-imines having mono- or di-substituent on a 1 and/or 2 position are preferred.

The dissolved copper can not be separated from the benzophenone-imine by filtration or centrifuging. The copper dissolved in a benzophenone-imine may be in the state of metallic copper, monovalent copper or divalent copper.

The materials which contain copper and can be used for preparing the catalyst of this invention are selected from metallic copper, copper alloy, copper oxides, copper hydroxide, copper carbonate, copper sulfide, formic acid salt of copper, salts of phenols and copper and mixtures thereof. Metallic

copper may be used as it is. Metallic copper supported on a carrier may be used.

Examples of the copper oxide include cuprous oxide and cupric oxide.

Basic copper carbonate is also included in the copper carbonate.

Examples of phenols constituting the salts of phenols and copper include phenols or substituted phenols, such as phenol, cresol, xylenol, trimethylphenol, catechol, resorcin, hydroquinone, aminophenol and hydroxybenzophenone.

The catalyst of this invention can be prepared by merely dissolving the copper-containing material e.g. metallic copper or specified copper compound (sometimes referred to as copper component) in a benzophenone-imine. However, when the solubility of copper component is a benzophenone-imine is not sufficient, or in order to increase dissolving speed, a mixture of copper component and a benzophenone-imine may be heated and/or stirred. Contact of the mixture of copper component and a benzophenone-imine with oxygen hydrogen or ammonia effectively accelerates dissolving of copper component in a benzophenone-imine, for example, in order to dissolve metallic copper in a benzophenone-imine, it is preferable to use oxygen. Whereas in order to dissolve cuprous oxide, cupric oxide, copper hydroxide or copper carbonate it is preferable to use hydrogen; and in order to dissolve copper hydroxide or copper carbonate, it is preferable to use ammonia. An inert gas, such as nitrogen, argon or helium may be used with oxygen, hydrogen or ammonia.

Air, carbon monoxide, carbon dioxide or water vapor may also be used under specific conditions in order to accelerate dissolving of copper component in a benzophenone-imine.

The amount of the catalyst employed in this invention is much less than the amount of catalyst employed in the prior art processes. The catalyst of this invention is used in an amount of 1/2000—1/100 mol per 1 mol of benzophenone-imine, preferably 1/1800—1/150 mol, and more preferably 1/1600—1/200 mol. Presence of undissolved metallic copper or copper compounds does not inhibit the reaction of the benzophenone-imine with molecular oxygen.

The reaction conditions under which the oxidation reaction of benzophenone-imine is carried out depend on activity of catalyst employed and state of the reaction system. In general, the reaction temperature is in the range of 60 to 300°C, preferably 70 to 250°C and more preferably 90 to 230°C. The reaction time depends on activity at the catalyst and the degree of conversion of benzophenone-imines. In general, the time is in the range of 0.1 hour to several tens of hours.

The molecular oxygen may include pure oxygen, oxygen enriched gas and mixture of oxygen and nitrogen, namely air. The reaction pressure may be reduced pressure, atmospheric pressure or super atmospheric pressure. In general, when oxygen alone is used as molecular oxygen, the reaction pressure is in the range of 1 to 20 atms. When air is used as molecular oxygen, the reaction pressure is in the range of 1—40 atms.

The way of introducing oxygen or an oxygen-containing gas into the reaction system is not critical. The oxygen gas or oxygen-containing gas may be introduced into the reaction system by blowing the gas into the liquid continuously, or it may be present as a super atmospheric gas in an enclosed reaction system containing the liquid. Preferably, the molecular oxygen is blown into the liquid in order to prevent hydrolysis of benzophenone-imines. It is preferable to use dry oxygen gas or dry oxygen-containing gas as molecular oxygen.

The reaction method may be continuous or batchwise. In order to increase the rate of reaction, it is preferable to use a multi-vessel continuous process, the so called cascade process.

The reaction of this invention proceeds without using any solvent. However, a solvent may be used in order to promote dissolution of the benzophenone-azines or to keep the reaction system in a solution state.

It is preferable to use a solvent which is not subjected to decomposition in the ammoxidation of benzophenones and in the oxidation of benzophenone-imines, and promotes dissolution of the benzophenone-azines formed by oxidation reaction of benzophenone-imines and which does not mix well with water. Examples of the solvents include benzene, toluene, o-, m- or p-xylene, ethylbenzene, mesitylene, cumene, pseudocumene, amylbenzene, aromatic hydrocarbons having 6—16 carbon atoms and mixtures thereof, chlorobenzene, o-, m- or p-dichlorobenzene, nitrobenzene, o-, m- or p-dinitrobenzene, o-, m- or p-chlorotoluene, diphenyl, phenanthrene, anisole, diphenyl ether, acetophenone, dibenzoyl, benzophenone, hexane, heptane, cyclohexane, cyclooctane, ethylcyclohexane, ethylene dichloride, tetrachloroethylene, diisopropyl ether, butyl acetate, butyl benzoate, phenyl benzoate, dimethyl phthalate, and pyridines.

It is preferable in the present invention to use benzophenone-imines obtained from benzophenones. In this case, the unreacted benzophenone may act as a solvent.

Although according to the present invention the amount of the catalyst employed is much less than that in the prior art processes, the reaction proceeds with a high selectivity and while maintaining high catalytic activity. In addition, since the amount of catalyst employed in this invention is very small, only a small amount of chemicals need be employed for recovering dissolved copper from the reaction system in which the reaction has been effected. For example, in case of recovering dissolved copper from the reaction system by extraction, the amount of a sulfuric acid solution, hydrochloric acid or ammonia solution employed for extraction is small. Furthermore, such recovering operation can be effected even by using small scale apparatus or facilities.

The present invention is further illustrated by the following Examples. However, this invention is not limited by these examples, and changes and modifications within the spirit and scope of this invention can be effected. Yield is by mol percent. In these examples, selectivity means ratio of mol of object product obtained to mol of raw material employed. The percents and parts on proportion of components are by weight, unless otherwise specified.

Example 1

Into a reactor was charged 30 grs. (imine 39.2 milli mol) of benzophenone-imine (benzophenone-imine 23.6%; the remainder was benzophenone). While blowing an oxygen gas, the solution was heated to 140°C. To the solution was added 1.5 grs. of commercially available powdered copper (produced by Wako Jun-yaku Co.). Thereafter, oxygen was blown from the bottom of the reactor at one atmospheric pressure for 2 hours at a rate of 100 ml/min. with stirring. Undissolved copper powder was removed by using a glass filter (filter size G—4). The filtrate was analyzed by gas chromatography. The analysis showed that benzophenone-azine was formed in yield of 28% (5.5 milli mol).

Colorimetry analysis showed that dissolved copper (250 ppm) was contained in the system.

Thereafter, the filtrate (imine:copper = 1:1/240 by mol) was charged into the reactor and then the reaction was continued for an additional 2 hours by blowing oxygen into the filtrate under the same conditions as above. The gas chromatography analysis showed that benzophenone-azine was formed in yield of 92% (18.0 milli mol). No by-products were detected.

Example 2

Into a reactor was charged 30 grs. (imine 39.2 milli mol) of benzophenone-imine (benzophenone-imine 23.6%; the remainder benzophenone). To the solution was added 1.5 grs. of commercially available powdered copper (produced by Wako Jun-yaku Co.). Thereafter, nitrogen was blown from the bottom at one atmospheric pressure for 1 hour at a velocity of 100 ml/min. with stirring. Undissolved copper powder was removed by using a glass filter (filter size G—4). The filtrate was analyzed by gas chromatography. The analysis showed that benzophenone-azine in amount of less than 0.1% was formed.

Colorimetry analysis showed that dissolved copper (77 ppm) was contained in the system.

Thereafter, the filtrate (imine:copper = 1:1/1080 by mol) was charged into the reactor. While blowing oxygen from the bottom of the reactor at one atmospheric pressure and a rate of 100 ml/min. the reaction was effected for 2.5 hours at 140°C. Gas chromatography analysis showed that benzophenone-azine was formed in a yield of 35% (6.9 milli mol).

Example 3

Commercially available powdered copper (produced by Wako Jun-yaku Co.) (2 g) was heated to 140°C in an electric furnace and then heat-treated for 3 hours while blowing air thereinto. Dissolving of copper in benzophenone-imine, introducing of nitrogen and filtering of undissolved copper was repeated as in Example 1 except that 1.5 g of heat-treated copper was used.

The filtrate (imine:copper = 1:1/300 by mol) was charged into a reactor. The reaction was effected in the same way as in Example 2. Gas chromatography analysis showed that benzophenone-azine was formed in yield of 97% (19 milli mol).

Example 4

Into a reactor were charged 30 g of benzophenone-imine (benzophenone-imine 23.9%; the remainder was benzophenone) and 0.5 g of commercially available powdered cupric oxide (Wako Jun-yaku Co.). When nitrogen was blown into the mixture at one atmospheric pressure at a rate of 100 ml/min., the mixture was heated to 140°C. When the temperature reached 140°C, supply of nitrogen was discontinued. The hydrogen was blown into the mixture for 40 minutes at speed of 200 ml/min with stirring.

Solid material containing undissolved copper component was removed from the solution by using a glass filter (filter size G—4). Colorimetry analysis showed that copper concentration in the filtrate was 700 ppm. Gas chromatography analysis showed that benzophenone-azine was formed in a yield of 0.3%.

Thereafter, the filtrate (imine:copper = 1:1/120 by mol) was charged into the reactor while blowing oxygen from the bottom of reactor at one atmospheric pressure and a rate of 100 ml/min. the reaction was effected for 1 hour at 140°C. Gas chromatography analysis showed that benzophenone-azine was formed in a yield of 30% (5.9 milli mol).

Example 5

Into a reactor was charged 40 g of benzophenone-imine (benzophenone-imine 23.0%; the remainder was benzophenone). The solution was heated at 140°C while blowing nitrogen thereinto. Cuprous oxide (2 g) (Wako Jun-yaku Co.) was added to the solution, and then the mixture was stirred for 2 hours. The solid material was removed from the solution by using a glass filter (filter size G—4).

Thereafter, the filtrate (imine:copper = 1:1/270 by mol) was charged into the reactor while

blowing oxygen from the bottom of reactor at one atmospheric pressure at a rate of 100 Nml/min. The reaction was effected for 2.0 hours at 140°C with stirring. Gas chromatography analysis showed that benzophenone-azine was formed in a yield of 80%.

Example 6

Into a reactor was charged 30 g (imine 41.8 milli mol of benzophenone-imine (benzophenone-imine 25.2%; the remainder was benzophenone). The solution was heated to 140°C while blowing oxygen into the solution. Copper formate, $Cu(HCOO)_2 . 2H_2O$ (21 mg) as a catalyst was added to the solution (molar ratio of imine:copper in the solution was 1:1/385) while blowing oxygen into the solution from the bottom of reactor at a rate of 100 ml/min. The reaction was effected at 140°C at one atmospheric pressure for 2.5 hours. Gas chromatography analysis showed that benzophenone-azine was formed in a yield of 85% (17.8 milli mol).

Example 7

Into a reactor was charged 30 g (imine 41.8 milli mol) of benzophenone-imine (benzophenone-imine 25.2%; the remainder was benzophenone). Copper hydroxide, $Cu(OH)_2$ (9.0 mg, 0.092 milli mol) was added to the solution while blowing an ammonia gas (20 ml/min.). The mixture was heated to 160°C and then was stirred for 1 hour to dissolve the copper component in benzophenone-imine.

While blowing oxygen into the solution from the bottom of reactor at a rate of 100 ml/min., the reaction was effected at 140°C at one atmospheric pressure for 1 hour. Gas chromatography analysis showed that benzophenone-azine was formed in a yield of 36% (7.5 milli mol).

Example 8

The procedure of Example 1 was repeated except that 30 g of 4-methyl-benzophenone-imine (4-methyl-benzophenone-imine was 20.0%; the remainder was 4-methyl-benzophenone) was used.

The filtrate was analyzed by colorimetry analysis. The yield of 4,4'-dimethylbenzophenone-azine was 25% (3.9 milli mol) and concentration of dissolved copper was 220 ppm.

The filtrate (imine:copper = 1:1/220 by mol) was charged into the reactor. While blowing oxygen at a rate of 100 ml/min. the reaction was effected for 2 hours with stirring. Gas chromatography analysis showed that 4,4'-dimethyl-benzophenone-azine was formed in yield of 93%.

Example 9

The filtrate was prepared by repeating the procedure of Example 1. The filtrate (imine:copper = 1:1/180 by mol) was charged into autoclave, and then was heated to 140°C. While blowing air under pressure of 4 $Kg/cm^2G$ into the mixture at a rate of 750 ml/min. (as calculated for 0°C and 1 atm), the reaction was effected for 2 hours. The yield of benzophenone-azine was 92%.

Example 10

Into a flask equipped with an agitator were charged 2.0 Kg of benzophenone-imine (benzophenone-imine 23.6%; the remainder was benzophenone) and 200 g of commercially available powdered copper (Wako Jun-yaku Co.). The reaction was effected at 140°C for 2 hours while blowing pure oxygen into the mixture. 45.3 percent of benzophenone-imine employed was converted to benzophenone-azine. The agitation was discontinued, so solid material was settled. The supernatant liquid was separated from the solid material. The supernatant liquid (concentration of imine = 12.9% by weight, dissolved copper = 305 ppm and imine:copper = 1:1/150 by mol) was used as a raw material.

The raw material (120 g) was charged into a stainless steel bubbling column having a sparger at its bottom, and then was heated to 130°C. Air under pressure was charged at a rate of 6N l/min. while maintaining the pressure of the column at 2 $Kg/cm^2G$. After the reaction was continued for 1 hour, the remainder of the raw material was charged at a rate of 40 g/hr by using a pump and the reaction product was discharged at a velocity of 40 g/hr from its bottom so as to keep the liquid level constant. Average residence time of the solution was 3 hours. Sampling was effected periodically and the sample was analyzed. The reaction was continued for 45 hours. The average yield of benzophenone-azine was 94.7%.

Control Test 1

The procedure of Example 6 was repeated except that commercially available cuprous chloride (Wako Jun-yaku Co.) was used. The molar ratio of imine to dissolved copper was 1:1/260. The yield of benzophenone-azine was 1.5% (0.3 milli mol).

Example 11

20 ml of an aqueous cupric formate solution containing 1.9 g of cupric formate was blended with 40 ml of ethanol solution containing 4 g (20 milli mol) of 2-hydroxybenzophenone at 60°C to form crystalline material. The crystalline material was removed by filtration, washed with water, and washed with ethanol, and then vacuum-dried, whereby copper (II) salt of 2-hydroxybenzophenone was obtained. The salt contained Cu 13.2%, C 68.5%, and H 4.15%.

The procedure of Example 6 was repeated except that 48 mg (0.100 milli mol) of the copper (II) salt of 2-hydroxy benzophenone prepared above was used. The reaction was effected for 2.5 hours. The yield of benzophenone-azine was 18% (3.8 milli mol).

Examples 12 and 13

The procedures of Example 6 were repeated except that each of catalysts shown in Table 1 was used. The results are shown in Table 1.

TABLE 1

| Ex. No. | Catalyst | reaction time | yield of benzophenone-azine |
|---|---|---|---|
| 12 | Cupric acetate, basic $Cu(CH_3COO)_2 \cdot CuO \cdot 6H_2O$ 18 mg (0.049 milli mol) | 1 hr. | 21% (4.4 milli mol) |
| 13 | Cupric sulfide 13 mg (0.136 milli mol) | 1 hr. | 18% (3.8 milli mol) |

## Claims

1. A process for producing a benzophenone-azine having the formula

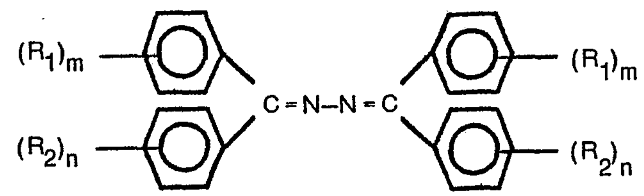

wherein $R_1$ and $R_2$ may be the same or different, and each of $R_1$ and $R_2$ are independently aliphatic, alicyclic or aromatic hydrocarbon having 1—10 carbon atoms,

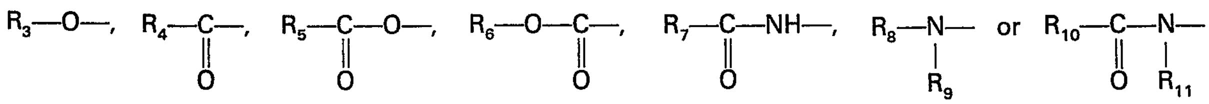

wherein $R_3$, $R_4$, $R_5$, $R_6$, $R_7$, $R_8$, $R_9$, $R_{10}$ and $R_{11}$ are independently alkyl having 1—10 carbon atoms; halogen; hydroxy; nitro; or cyano; or $R_1$ and $R_2$ when taken together form a single bond or substituted or unsubstituted methylene wherein the substituent is methyl; m is 0 or integer of 1—5; and n is 0 or integer of 1—5, which comprises

reacting a benzophenone-imine having the formula

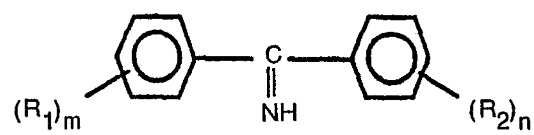

wherein $R_1$, $R_2$, m and n are as defined above with molecular oxygen, characterised in that

the reaction is carried out in the presence of a catalyst composed of dissolved copper in an amount 0.0005—0.01 mol per 1 mol of the benzophenone-imine in terms of copper atom, wherein the dissolved copper is obtained by dissolving in the benzophenone-imine at least one copper-containing material selected from metallic copper, copper alloys, copper oxides, copper hydroxide, copper carbonate, copper sulfide, formic acid salt of copper, salts of phenols and copper, and mixtures thereof.

2. The process as defined in Claim 1 wherein the dissolved copper is obtained by contacting said copper-containing material with a gas selected from oxygen, oxygen-containing gas, hydrogen, hydrogen-containing gas, ammonia, carbon monoxide, carbon dioxide and water vapour.

**Revendications**

1. Procédé de production d'une benzophénone-azine ayant pour formule

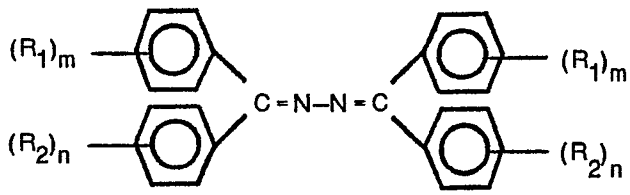

où $R_1$ et $R_2$ peuvent être identiques ou différents, et chacun de $R_1$ et $R_2$ est indépendamment un hydrocarbure aliphatique, alicyclique ou aromatique ayant de 1 à 10 atomes de carbone,

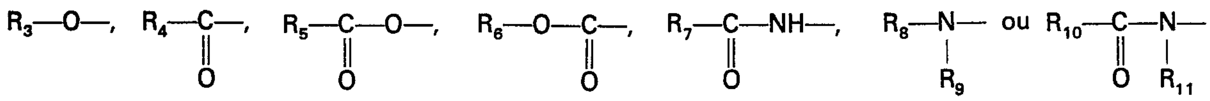

ou

où $R_3$, $R_4$, $R_5$, $R_6$, $R_7$, $R_8$, $R_9$, $R_{10}$ et $R_{11}$ sont indépendamment un alcoyle ayant de 1 à 10 atomes de carbone; un halogène; un hydroxy; un nitro; ou un cyano; ou bien $R_1$ et $R_2$ quand ils sont pris ensemble forment une simple liaison ou un méthylène substitué ou non substitué où le substituant est du méthyle; m est zéro ou un nombre entier de 1 à 5 et n est zéro ou un nombre entier de 1 à 5 qui consiste à:

faire réagir une benzophénone-imine ayant pour formule

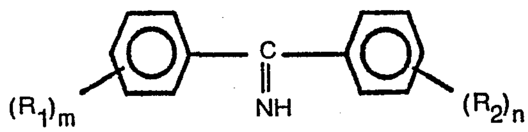

où $R_1$, $R_2$, m et n sont tels que définis ci-dessus, avec de l'oxygène moléculaire, caractérisé en ce que la réaction est effectuée en présence d'un catalyseur composé de cuivre dissous en une quantité de 0,0005—0,01 mole pour 1 mole de la benzophénone-imine en termes d'atome de cuivre, le cuivre dissous étant obtenu en dissolvant, dans la benzophénone, au moins un matériau contenant du cuivre choisi parmi du cuivre métallique, des alliages de cuivre, des oxydes de cuivre, de l'hydroxyde de cuivre, du carbonate de cuivre, du sulfure de cuivre, le sel de cuivre de l'acide formique, des sels de phénols et de cuivre et leurs mélanges.

2. Procédé selon la revendication 1, caractérisé en ce que le cuivre dissous est obtenu par contact du matériau contenant du cuivre avec un gaz choisi parmi l'oxygène, un gaz contenant de l'oxygène, de l'hydrogène, un gaz contenant de l'hydrogène, de l'ammoniac, de l'oxyde de carbone, du gaz carbonique et de la vapeur d'eau.

**Patentansprüche**

1. Verfahren zur Herstellung eines Benzophenonazins der Formel:

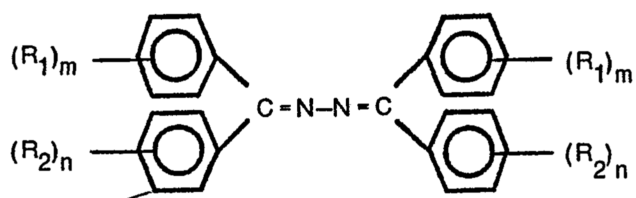

worin bedeuten: $R_1$ und R ... oder verschieden sein können, einzeln jeweils einen aliphatischen, alicyclischen oder ... enwasserstoffrest mit 1—10 Kohlenstoffatomen, einen Rest der Formeln:

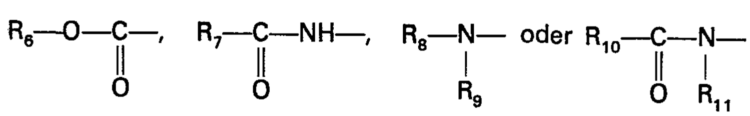

oder

in welchen $R_3$, $R_4$, $R_5$, $R_6$, $R_7$, $R_8$, $R_9$, $R_{10}$ und $R_{11}$ einzeln für einen Akylrest mit 1—10 Kohlenstoffatom(en) stehen, ein Halogenatom, einen Hydroxyrest, einen Nitrorest oder einen Cyanorest, oder zusammen eine Einfachbindung oder einen gegebenenfalls methylsubstituierten Methylenrest; m = 0 oder eine ganze Zahle von 1—5 und n = 0 oder eine ganze Zahl von 1—4, durch Umsetzen eines Benzophenonimids der Formel:

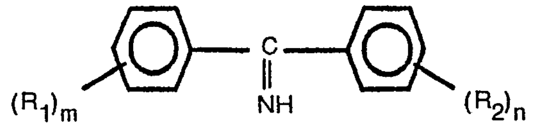

worin $R_1$, $R_2$, m und n die angegebene Bedeutung besitzen, mit molekularem Sauerstoff, dadurch gekennzeichnet, daß man die Umsetzung in Gegenwart eines Katalysators in Form von gelöstem Kupfer, das durch Auflösen mindestens eines kupferhaltigen Materials, bestehend aus metallischem Kupfer, einer Kupferlegierung, einem Kupferoxid, Kupferhydroxid, Kupfercarbonat, Kupfersulfid, einem Kupfersalz der Ameisensäure, einem Salz aus einem Phenol und Kupfer oder einer Mischung derselben in dem Benzophenonimin erhalten wurde, durchführt, wobei die Katalysatormenge 0,0005—0,01 Mole (ausgedrückt als Kupferatom) pro 1 Mol Benzophenonimin beträgt.

2. Verfahren nach Anspruch 1, dadurch gekennzeichnet, daß man das gelöste Kupfer durch Inberührungbringen des kupferhaltigen Materials mit einem aus Sauerstoff, einem sauerstoffhaltigen Gas, Wasserstoff, einem wasserstoffhaltigen Gas, Ammoniak, Kohlenmonoxid, Kohlendioxid und Wasserdampf bestehenden Gas herstellt.